# EUROPEAN PATENT SPECIFICATION

(11) **EP 4 463 875 B1**
(45) Date of publication and mention of the grant of the patent: **05.08.2026**
(21) Application number: 23700921.2
(22) Date of filing: 09.01.2023
(51) Int. Cl.: G16H 40/60, A61B 34/00

(54) **ANGIOPLASTY SYSTEM**
ANGIOPLASTIESYSTEM
SYSTÈME D'ANGIOPLASTIE

(30) Priority: 13.01.2022 ES 202230044 U
(43) Date of publication of application: 20.11.2024
(73) Proprietor: Martinez Marín, David, 03202 Elche (ES); Fundación Universitas Miguel Hernández De la Comunitat Valenciana, 03202 Elche (Alicante) (ES)
(72) Inventor: MARTINEZ MARÍN, David, 03202 Elche (ES)
(74) Representative: Ibarra Garcia, Isabel
(86) International application number: PCT/EP2023/050295
(87) International publication number: WO 2023/135071

(56) References cited:
- EP-A2- 0 581 708
- WO-A1-2014/025394
- WO-A1-2016/206975
- WO-A1-2021/194872
- WO-A1-2021/243311
- US-A1- 2013 282 084
- US-A1- 2020 094 018

## Description

### Technical Field

The present invention relates to the technical field of the material used for the angioplasty or dilation process of a blood vessel presenting stenosis. More in particular, the present invention concerns an angioplasty system. The invention may also refer to a procedure and to an inflation device for angioplasty; for example, a balloon or balloon-mounted stent, provided with, in particular, automatic adjustment and artificial intelligence.

### Background of the Invention

Currently, the catheter-based technique for the treatment of narrowed vessels either consists in the dilation of the vessels by means of a ball or balloon or by means of a balloon-mounted stent. Said procedure is known as Percutaneous Transluminal Angioplasty (PTA).

It can be said that PTA consists of the inflation, plateau or stable, and deflation phases, and, for the pressure delivery thereto, the most commonly used device is a manual pressure syringe with a pressure gauge which allows the operator to visually control the inflation pressure of the balloon. In the inflation phase, the pressure increases. In the plateau phase, the nominal pressure is reached according to the specifications of the angioplasty device (balloon or balloon-mounted stent). Finally, during the deflation phase, a reduction of balloon pressure is observed.

Amongst the adverse results of these types of treatments we find the elastic withdrawal (or recoil), the vessel dissection and the restenosis.

The elastic recoil is the return to the initial state. In turn, the dissection consists of the partial fragmentation of the diseased arterial wall and its detachment towards the vessel lumen against the flow wave. The total inflation time is important for the healing of the vessel from the barotrauma leading to said dissections. At the same time, a residual stenosis or insufficient lumen gain may occur, due to an insufficient pressure and/or treatment time. Finally, the restenosis, due to a scarring reaction towards the vessel lumen, may entail the need for new treatments.

Faced with the difficulty in identifying the variables causing the elastic recoil and the dissection, the interventionist community has opted, to a large extent, for the mechanical support by means of a stent implant. This solution was mainly designed for angioplasty treatments in coronary vessels.

However, in the arteries of the lower limbs, due to stents not working correctly, the employed solution consists in the use of a stentless inflation balloon.

With the sufficiently developed architecture of balloon technology, the research groups focus on how to deliver the necessary pressure to the vessel to overcome the stenosis.

Therefore, the inflation and deflation speeds, the total treatment time, and the maximum inflation pressure are variables that are to be considered nowadays, so that the angioplasty must necessarily be carried out precisely, reproducibly and in a way liable to revision. To that end, computer-assisted inflation solutions have been proposed. The end is to standardise the PTA procedure and make it reproducible trying to avoid the variability of the manual treatments.

These computerised solutions are assisted by a pressure-motorised pump and are programmable in all treatment phases. However, they exhibit a great disadvantage in that, once the treatment parameters are entered and the treatment has started, their modification is not allowed without interrupting the treatment automation.

Other authors have proven how the behaviour of the pressure-volume curve or the one based on the pressure-diameter curve offers sufficient information, and in real time, to explain and predict the desirable or undesirable events that occur from the start of the inflation phase to the end of the plateau.

Therefore, the impossibility of controlling the variables, and the hardly -known events that occur during the PTA, together with other disadvantages, has not allowed the development and the generalisation of these computer-assisted inflation devices described over 20 years ago.

Documents US2020094018 and US2009312740 can be mentioned as examples of the state of the art.

In document US2020094018, treatment programs are defined which include expansion-contraction cycles in a predefined frequency profile, configured to treat a particular condition, such as the calcification of an arterial duct, and expansible intraluminal systems, methods and devices are described, associated to achieve an improved tissue response to dilation or other tissue expansion procedures. In this case, however, the device is configured to cause, by means of previous programming, a stretching or dilation in mammal tissues and/or vessels with a cyclic pattern based on the Mullins effect, but the behaviour of the diseased vessel during its dilation by means of an angioplasty device with or without a stent does not only depend on the named Mullins effect, and this device is not able to cope with the different situations that can occur, since it responds to a fixed and previously predefined programming, in all its phases, before starting each treatment.

Document US2009312740 defines a method implemented by a motorised inflation device to prepare a balloon catheter for its use during a medical procedure. The method includes drawing an amount of medical fluid from a fluid reservoir to the inflation device during a first motorised operation thereof, eliminating an amount of air from the balloon catheter during a second motorised operation of the inflation device, and injecting the medical fluid amount thereof in the balloon catheter during a third motorised operation to inflate a balloon located in a distal end of the balloon catheter. In an embodiment, the motorised inflation device is coupled to an angiographic injector system, and the balloon inflation device and the injector system may be controlled by means of a common control panel, or console. Likewise, the system is programmable based on an input formed by a feedback loop of the pressure or pressure variation data. Thus, these loops are sudden pressure drops that exist during the procedure and which are due to the build-up of yielding plaque, which is the ultimate objective of the procedure. In this case, the computer is programmed to be able to detect, thanks to these sensors, the feedback information that causes it to carry out a "desired" action and, therefore, defined before the start of the treatment, like, for instance, deflating the balloon or maintaining the balloon inflated during a period of time.

The current technological developments allow for a greater calculation capacity, connectivity and machine learning, all of which may be the response to the complexity of the disease and to the behaviour exhibited by the vessels and the tissues, diseased or not, when they are subjected to high pressures during these types of treatments.

It would be a great step forward for the vascular surgical intervention techniques to apply these developments and to be able to use a system which allows a complete and intelligent mechanisation of a therapeutic procedure, acting within safety limits, and, at the same time, to be capable of ensuring the best possible result of the treatment.

Document US2013/282084A1 discloses a catheter and catheter system can use energy tailored for remodelling and/or removal of target material proximate to a body lumen, often of stenotic material or tissue in the luminal wall of a blood vessel of a patient.

Document US2020/094018A1 discloses treatment programs utilizing expansion elements, such as those associated with occlusion and therapeutic agent delivery devices, systems, and methods.

### Disclosure of the Invention

To that end, embodiments of the present invention provide, according to a first aspect, an angioplasty system according to claim 1.

The proposed system comprises an inflation device for the inflation of an angioplasty device within a blood vessel presenting stenosis. In particular, the inflation device comprises a reservoir of contrast fluid with a pressure-adjusting device; a connector for the connection to the angioplasty device which is connected to the reservoir through disposable tubing; a pressure sensor arranged inside the reservoir; and an electronic-actuation valve for controlling the ingress or release of the contrast fluid. In addition, the inflation device comprises safety means for the emergency deflation of the angioplasty device; powering means; and a digital interface, for example, for the entry of values/data and the machine-learning algorithm.

In turn, the inflation device comprises a microprocessor connected to the electronic-actuation valve and to the pressure-adjusting device for controlling the operating parameters of the inflation device and comprising a first operation software with at least one operational application for remote management; a memory connected to the microprocessor; and connection means of the first operation software to the Internet or an intranet.

The system further comprises a second artificial intelligence software for the automated self-learning of the inflation device and which includes connection means thereof to the Internet and which is operatively connected to the first operation software; a database for storage of data or treatment models which is operatively connected to the Internet or an intranet and which includes a management software; and a radiological arch to capture/acquire and process images of the blood vessel or of the area where the blood vessel to be treated is located, and also of the volume of contrast fluid circulating therein.

In particular, the microprocessor is operatively connected to the radiological arch and to the database, such that the inflation device may carry out a learning process to operate autonomously and adjust itself automatically, using previous data or treatment models.

According to the present invention, the second artificial intelligence software may comprise, for instance, neural networks, deep learning algorithms, clustering algorithms and/or decision trees, etc. This second software may be implemented/run in the radiological arch itself (that is, in the arch console), in the inflation device itself (that is, by the microprocessor) or in a computing device, remote or independent from the radiological arch and from the inflation device. In particular, the second software is used to obtain the data or treatment models which are stored in the database. To that end, the second software uses the information, or part thereof, processed by the radiological arch, operational data from the inflation device itself, and, if available, data from the database itself.

In some embodiments, the system also comprises contrast fluid heating means.

In some embodiments, the pressure adjustment device comprises a motorised pump. In some embodiments, the safety means for the emergency deflation comprise a vacuum reservoir connected to the reservoir. Additionally, the safety means may also include manual safety means.

In some embodiments, the disposable tubing connected to the reservoir comprises two or more branches, each branch including a connector for its connection to a different angioplasty device.

In some embodiments, the powering means comprise a battery or a connection to the electric grid.

In some embodiments, the microprocessor connection means comprise a connection interface installed on the inflation device or a connection to an external electronic device running a corresponding application.

In some embodiments, the digital interface also comprises communication ports for data entry by means of a QR code reader or wirelessly.

Embodiments of the present invention provide, according to a second aspect, an angioplasty procedure which comprises: providing an inflation device like the one previously described; providing a database for the storage of data or treatment models, the database being operatively connected to the Internet or an intranet and including a management software; providing a second artificial intelligence software; acquiring and processing, by means of a radiological arch operatively connected to the microprocessor, one or more images of the blood vessel or of the area where the blood vessel is located; and operating and adjusting the inflation device autonomously and automatically according to previous data or treatment models, stored in the database, and obtained by running the second artificial intelligence software on, at least, the data processed by the radiological arch and on the data from the inflation device obtained by the first software.

Embodiments of the present invention also provide, according to a third aspect, an inflation device for the inflation of an angioplasty device within a blood vessel presenting stenosis. The device of the third aspect may include the different elements and software(s) previously described in the first aspect of the invention.

Therefore, the present invention, by allowing for the automatic adjustment of the inflation device and including artificial intelligence, significantly improves on the devices of the state of the art. This is due to the fact that, with the inflation device, it is possible to carry out the angioplasty procedure completely autonomously and, thanks to the artificial intelligence, without the need of taking into account the large number of unknown variables involved. The existence of these variables is the reason why the current programmable and automatic devices have not been accepted in the practice amongst therapists despite the precision and reproducibility they show. With the proposed inflation device, it is possible to omit all those variables to focus on identifying the successful treatment patterns and, this way, to precisely re-apply them when facing identical circumstances in future treatments.

To achieve the automatic adjustment during treatments, the pressure, volume and diameter behavioural changes during the inflation, the plateau phase and, finally, during the deflation are analysed and responded to.

The actions that trigger an automatic adjustment of the inflation device are a purging of air, a rupture of the angioplasty device, an obstruction and a pressure drop resulting from abrupt strain of the vessel. The purging of air makes it possible, since the air and any gas are compressible, for the inflation device to be capable of detecting the existence of gas due to volume and pressure changes. Following the refilling of the syringe with liquid and the closure of the ingress and egress system, the inflation device carries out a filling and/or emptying attempt. Without air in the system, a change of pressure and not of volume occurs, whereas, with air in the system, a volume change with a pressure change occurs. Automatically, the inflation device requests a change of position, to then evacuate the air and perform the verification again until no gas remains in the system.

In reference to the rupture of the angioplasty device resulting in an abrupt pressure drop, the inflation device, automatically, tries to compensate for it by increasing the volume. If there is no response by way of pressure restoration, a leakage exists and the inflation device stops the treatment and warns about the incident.

In turn, the obstruction implies an impossibility of volume change with increase or decrease of the pressure without having overcome the nominal pressure of the angioplasty device or having effected the deflation. And, faced with a pressure drop resulting from an abrupt strain of the vessel, the inflation device continues or resumes the inflation until reaching and maintaining the nominal inflation pressure of the device.

The inflation device is totally automatic, so that, once the procedure starts, this is carried out without intervention by the operator. However, it is possible that in certain circumstances it is programmed to request confirmation from the operator in some treatment phases. Thus, the present invention allows the inflation device to automatically adjust itself based on the initial data obtained in the pretreatment, as well as on the data obtained during the procedure and in real time. In addition, it is endowed with artificial intelligence, because, due to its connection with the radiological arch that captures and processes the images, it is to receive the feedback information needed for its machine learning. This connection to the arch allows it to be capable of carrying out a machine-learning process and being endowed with artificial intelligence.

The image processing allows to obtain an anatomic reconnaissance of the area to be treated, because the anatomic bone structures are characteristic of each area and are not radiotransparent, so that the device will be able to recognise them at all times thanks to the connection of the microprocessor to the radiological arch once the machine learning has allowed it to learn to identify them.

Based on the area to be treated, there can be different sets of implications, since the pathology often presents patterns which repeat within the variability that exists between patients. This is an information of great importance for the machine learning of the inflation device.

Another aspect that is achieved is the measurement of the vessel lumen, since, following a first acquisition and automatically, the inflation device will be capable of identifying the lumen areas of greater or lower diameter and the length of the stenoses. This information is of great importance for the correct choice of the inflation device according to the diameter and the length, as well as for the analysis of the results during the machine learning of the inflation device.

The following parameter that is achieved is the measurement of flow, since, knowing the diameter of the angioplasty device, knowing the volume of contrast delivered and the parameters of digital subtraction (images per second, number of pixels, etc.), it is possible to calculate the flow through the area of interest of the vessel before and after the treatment. A significant increase of the flow following the treatment, the latter being uniform along the treated vessel, implies a positive reinforcement, so that the treatment pattern is recalled and used in future identical, or even similar, cases. An insufficient increase of the flow causes the inflation device to recommend further treatment or that the therapist should assess alternative treatments.

The parameters obtained during the processing of the images, as well as the data learned by the device, may be shared and will be stored in said database. The health personnel will thus benefit from the most successful treatment patterns that are eventually added to the database following the machine learning of the inflation device. This way, and without knowing their origin other than the database itself linked to the inflation device, they all have access to the best and most successful treatment patterns for each circumstance.

To start with, and before the system/device itself has enough data of its own about patients and about treatments carried out, there may be a learning period during which the vascular interventionist community may cooperate.

In some exemplary embodiments, the database may be located in the cloud and use or be managed with Big Data technology. From the data available in the database (that is, the procedures carried out previously) treatment curve models will be generated for all possible cases from which the microprocessor of the inflation device will choose the best one for each new case when the treatment conditions of a specific model among those available are repeated.

With a sufficient build-up of data coming from treatments carried out and successfully completed, it ends up being included in the database. The end is to obtain an ideal pressure/diameter or pressure/volume treatment curve for each case and for each circumstance. In some exemplary embodiments, the data or treatment models carried out and completed may be stored in the memory of the inflation device so that the microprocessor may locally choose the most adequate one, without the need to interact with the database.

Thus, it is possible to obtain an inflation device for angioplasty treatment devices by means of which a greater actuation precision is achieved and with which it is possible to act on the patient according to some data obtained from experience and, above all, to modify the type of intervention based on the variability of this data which can occur during the execution of the angioplasty process itself depending on the particular variables of the patient and the stenosis he or she presents.

Since the tissue of the vessels is irregular and has parts that are softer, more or less calcified, more or less affected by the disease, etc. in practice and facing the same stenosis characteristics, the tissue may act differently, possibly behaving adequately during and following the angioplasty or, on the contrary, rupturing, narrowing again following the removal of the angioplasty device or suffering other reactions that entail the need for new treatments. With the inflation device any alteration in the normal inflation, plateau and deflation procedure is detected by the software as abnormal, and the device is able to react automatically, by means of a microprocessor command that rectifies the treatment, seeking the most adequate option to resolve said abnormality based on experience with other similar cases. Additionally, thanks to the data obtained from experience, it is possible to control the values of air pressure, inflation, plateau and deflation times, the speed with which each of said states is obtained, etc. Currently, it is not possible to control all these values, so that, although programmable or automatic devices may exist, none of them is able to react and adjust itself automatically when faced with a variation in the patient conditions. On the other hand, the inflation device proposed herein is able to adjust the treatment conditions once it has started.

On the other hand, the present invention solves the existing problem when carrying out angioplasty treatments in lower limb vessels, since it will provide a lot of information on the behaviour of the arteries and thus it will be possible to control the maximum treatment time necessary without negatively affecting the vessel. Likewise, it will be possible to determine the optimum pressure of the treatment for each case, without needing to reach the nominal pressure, by weighting the importance of other variables such as the total time and of each one of the phases, the treatment duration, the gained area and diameter, the difference between the maximum diameter before the deflation and the final diameter of the vessel, the difference between the pressure gradient before and after the treatment, or of the dynamic images obtained before and after completing the treatment, or of the information obtained with ultrasound of the spectral analysis of the flow curve before and after the treatment.

### Brief Description of the Drawings

The foregoing and other characteristics and advantages will be more fully understood from the following detailed description of some exemplary, merely illustrative and nonlimiting embodiments, with reference to the attached drawings, wherein:
Fig. 1shows a schematic view of an angioplasty inflation device with automatic adjustment, according to an embodiment of the invention.
Fig. 2 schematically shows an embodiment of the proposed system.

### Detailed Description of the Invention and of Some Exemplary Embodiments

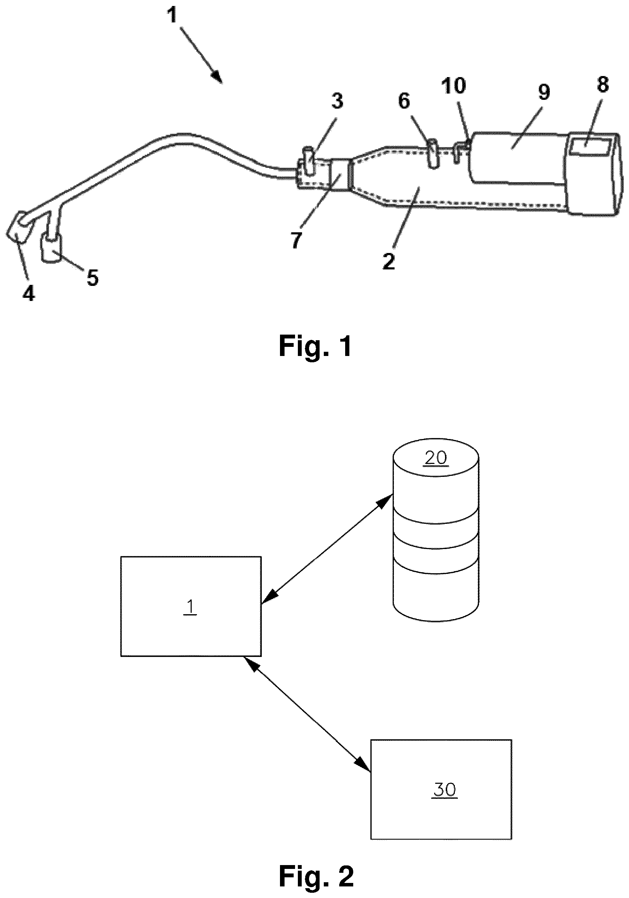

Fig. 1shows a preferred embodiment of the inflation device (1) of the present invention. The inflation device (1), or simply device (1) is used/cooperates with a database (20) and with a radiological arch (30), see Fig. 2.

The device (1) of this example, as seen in the figure, comprises a reservoir (2) of fluid contrast with a pressure-adjusting device (3), formed by a motorised pump, for example; two connectors (4, 5) each for connecting to an angioplasty device and connected with the reservoir (2) through disposable tubing, a pressure sensor (6), and an electronic actuation valve (7) for controlling the ingress or release of the contrast fluid.

With the inclusion of the two connectors (4, 5), the device (1) allows to safely treat vessels which currently present a lot of complications. Thus, for example, when angioplasties must be carried out in complicated areas, as can be the case of a vessel presenting stenosis, but which is too close to another vessel, the dilation of the vessel to be treated by means of the inflation of an angioplasty device within it may affect the neighbouring vessel, as it can cause the shifting of the plaque towards it and its occlusion. Currently, to solve this problem it is necessary to involve three people, one of them inflating a first angioplasty device in a first vessel, another inflating a second angioplasty device in a second vessel and a third person holding both angioplasty devices. This way of proceeding is very complicated, as it requires a perfect synchronisation between all three people participation in the process and it involves a large dependence on the human factor in such a delicate process. Therefore, with the device (1) it is possible to automatically carry out the inflation of both angioplasty devices, controlling the values of both and instantly reacting to any unforeseen event which may occur in either angioplasty treatment. Device (1) is capable of resolving any of these unforeseen events by modifying variables such as the inflation time, pressure and/or volume and the steps to be followed, based on the behaviour of each of the vessels. In any case, it must be noted that, in other examples, not illustrated, the device (1) comprises a single connector.

The device (1), in turn, comprises safety means for the emergency deflation of the angioplasty device(s). In this preferred embodiment of the invention, these means are formed by a vacuum reservoir (9) connected to the reservoir (2) which will be actuated by means of a safety valve. In addition, in this embodiment, the safety means comprise manual safety means (10) for the release of the pressure of the pressure fluid. In other embodiments, there may be a single type of safety means.

The device (1) also comprises a digital interface (8), for example, for the entry of data/values. This digital interface (8) allows for the manual entry by the operator of the treatment parameters. In this preferred embodiment of the invention, the device also comprises communication ports for data entry by means of a QR code reader or wirelessly.

The device (1) also comprises powering means thereof, in this case formed by a connection to the electric grid. However, in other embodiments they may be formed by a battery.

The device (1) also consists of a microprocessor for controlling the operating parameters thereof and a memory connected to the microprocessor. The microprocessor is connected to the electronic actuation valve (7) and to the pressure-adjusting device (3), and comprises a first operation software with at least one operational application. Connection means of the first operation software to the Internet or an intranet are also included. The microprocessor is connected to the radiological arch intended to carry out the acquisition and processing of the images of the area to be treated.

In this preferred embodiment of the invention, the connection means of the first operation software to the Internet or an intranet are formed by a connection interface installed on the device (1). However, in other embodiments, these connection means may be formed by a connection to an external electronic device presenting a corresponding application.

In some exemplary embodiments, the behaviour of the pressure/volume curve provides the information necessary to carry out the automatic adjustment of the device (1). Based on the initial parameters, the microprocessor may choose a treatment curve model from among those available in the database, in particular the one having the best historical result.

During the treatment, the automatic adjustment of the device (1) consists in that, based on the behaviour of the pressure/volume curve, the microprocessor may choose, for example, among continuing with the initial model, automatically changing the model, requesting confirmation from the operator to continue or proceeding with the deflation and interrupting the treatment.

Also included is a second artificial intelligence software, with connection means thereof to the Internet or to the first operation software, for the self-learning of optimal processes. This second software, during the initial usage phase of the device (1), will have a primary function so that the device (1) may extract data and conclusions from all previous experiences collected in the database, located particularly in the cloud. In any case, once the device (1) itself already contains all information relative to said experiences, if so desired, may stop resorting to the information contained in the database, since it is already at its disposal in said memory, and, therefore, it is already available to the first operation software itself.

In some examples, the device (1) may include heating means of the contrast fluid.

In reference now to the angioplasty procedure, in an exemplary embodiment, it comprises the following steps. Firstly, a pretreatment is carried out for obtaining the stenosis characteristics in a blood vessel of the patient, and thereafter a treatment including the inflation, stable or plateau, and deflation steps of the device (1) within said vessel is carried out. The pretreatment known in the state of the art, consists in a premedication with anticoagulation and anti-aggregation at the operator's discretion. Next, an entry port is made for devices or a short or long guiding sheath, but with its end located as close as possible to the stenosis or occlusion to be treated.

The choice of the stenosis or stenoses or occlusion to be treated depends on the operator. A system is needed to objectively measure the result of the PTA during the procedure. In addition, since unfavourable premature results exist, but not immediate or deferred ones, said measuring system has to be reproducible. Thus, three methods may be carried out for the measurement of the stenosis. The first of them consists in a measurement of the pressure gradient, but has disadvantages due to the need to carry out a second puncture to house a measuring catheter distally to the lesion, in addition to the one necessary for the treatment, from which pressure is measured proximally or on the side of the lesion closest to the guiding sheath. A second method, this one being the one most commonly used, consists in the anatomical gradation of the stenosis following the injection of contrast and the digital subtraction angiography. It is necessary to anatomically reference and register the location of the lesion. The choice of a long angioplasty balloon is frequent in the case of multiple stenoses in tandem, as well as for the treatment of a single but long lesion. In addition to the percentage of greater narrowing, the length of the lesion has to be taken into account. Images are obtained in at least two projections before and after the PTA and also while it is being carried out, with the balloon inflated. A second method is used with the inflation device of this preferred embodiment of the invention, due to its capacity to receive information from the radiological arch, which obtains and processes the images. A third method is the one using intravascular ultrasounds (IVUS) which allows both an anatomical and haemodynamic gradation, the latter being indirect. Because of this, it is an attractive method, although it has to be validated to serve as the only measuring method of the result.

In either case, general and specific images and series of images of the area to be treated are recorded.

Once the pretreatment has been carried out and the data necessary about the stenosis of the patient has been obtained, the angioplasty procedure is executed using the device (1). This procedure has a series of phases laid down next.

A first, capturing phase by means of the device (1) of the information relative to the anatomy and the haemodynamics from the digital subtraction arch (anatomical area, inner diameter of the vessel, length of the lesion, flow through the vessel and total flow in the area being studied). A second, connection phase of the device (1). Next, a third, filling phase of the reservoir (2) takes place with an adequate fluid and the purging of air.

The below process is followed, in particular, for the purging of air:
- The check list of the necessary previous steps is verified by means of the digital interface (8), and information is requested.
- The therapist submerges the extended end for the filling of a container with the desired contrast fluid. Said action is confirmed and the device (1) proceeds with the automatic filling of the reservoir (2) by means of the counter-clockwise movement by the motor of the piston up to its maximum capacity.
- At this time, the motor begins a short counter-movement, that is, clockwise, to remove the air that may have been introduced, discarding a small volume of fluid.
- The device (1) asks the therapist to mechanically close the ingress and egress of the contrast fluid and begins the verification of the presence of gas in the system. With a small recoil or filling movement of the piston, a pressure gauge detects if there are pressure changes, while volume changes take place or not with different scenarios.
   ✔ If the pressure decreases and the volume increases in the reservoir (2), it means that the system is airtight and there is gas inside.
   ✔ If the pressure is stable and the volume increases in the reservoir (2), it means that the system is not airtight and fluid and/or gas is coming in.
   ✔ If the pressure decreases without changes in the volume, it means that the system is airtight and no gas exists therein.
- The device (1) repeats the procedure until achieving the air tightness without the presence of gas therein.

Next, a fourth verification phase is carried out of the correct positioning of the angioplasty device(s). The anatomy has been previously examined and data has been captured relative thereto and to the haemodynamics or the flow through the diseased vessel and the area being studied. At this point, the ratio between the length of the device (1) and the length of the diseased segment of the vessel to be treated is important.

Once this verification has been carried out, a fifth, calculation phase by the device (1), both of the inflation rate based on the ratio between the pressure increase and volume, and of the deflation rate. Likewise, the device (1), thanks to its connection with the radiological arch, may also measure the diameter of the angioplasty device which is being inflated with radiological contrast at different pressures until reaching the nominal pressure, and further relating it to the delivered volume. In real-time analysis, curves relating these treatment parameters are generated: pressure - diameter, pressure - volume and volume - diameter.

Finally, the procedure has a sixth, automatic adjustment phase of the inflation device (1) based on the events which take place as the vessel is dilated (fractures, sliding of the plaques, strain of the outer layers and surrounding tissue, elastic recoil). This automatic adjustment phase, in particular, is carried out as follows:
- The inflation of the angioplasty device is started and there are no changes until reaching a certain pressure P1. If the procedure is suspended at this time a situation will be reached that is identical to the initial one.
- Having reached pressure P1, the plaque breaks and shifts slightly and settles, leaving space for the balloon or angioplasty device being inflated. If the procedure is stopped at this point, a dissection or separation of the plaque invading the vessel lumen is obtained without having obtained an inner diameter gain thereof. In the pressure-volume curve, this translates into a sawtooth with verticalization of the curve and then a small pressure drop while the volume increases. Therefore, the device (1) is capable of identifying the event and registers it to include it in the relevant information for its machine learning.

- Having reached a greater pressure or P2, the strain of the outer layers of the vessel and the surrounding tissues starts. It is reflected by a flattening of the pressure-volume curve which repeats constantly at a similar pressure, since it is an inevitable and necessary event. The device (1) includes the corresponding information for its machine learning. If the inflation is stopped at any point between P2 and the start of the plateau phase, a lesser or greater, but transitory, lumen gain is obtained as a pressure P3 greater than the strain limit of the outer layers of the vessel and of the surrounding tissues has not been reached during sufficient time in the plateau phase.
- Having reached a pressure P3 close to the nominal pressure of balloon inflation and maintaining it during the entire plateau phase, it is possible to overcome the strain limit of the outer layers and of the surrounding tissues. If deflation is effected before the end of a sufficiently prolonged plateau phase, it is likely that the elastic withdrawal or recoil will also occur due to haematoma caused in the outermost layer of the vessel. In this case, some "healing time" is required for said hematoma to stop, spread and not to cause compression on the recently gained lumen once the balloon is deflated and during the minutes and hours thereafter. The greater duration of the plateau phase favours the success and the durability of the procedure, although within limits, due to the possibility of causing thrombosis due to the prolonged stagnation.
- During the plateau phase, it may happen that, as the yield of the outer layers of the vessel and the surrounding tissues is achieved, thereby destructuring themselves and losing their elastic capacity, small pressure drops will take place. This phenomenon occurs even when the angioplasty device is a balloon-mounted stent, having been shown to have, when it happens, a negative repercussion on the treatment results. The device (1) is able to detect pressure variations of 0.05 atm, so that, in a programmed manner and in case a pressure drop occurs in this case, it proceeds to immediately correct said situation by resetting the chosen pressure for the plateau phase. The device (1) is capable of carrying out this correction with much greater sensitivity, speed and precision than those of human beings such as currently done.
- The deflation speed is also important when it comes to the result of these types of interventions. Counterintuitively, for yet unknown reason, a fast deflation causes fewer dissections than a slow deflation. The device (1) may "learn" the deflation pattern that generates the best result following training by means of a supervised machine learning. In this and other cases, the device (1) also serves as an aid in furthering the knowledge of the pathophysiology involved in the angioplasty process or intraluminal balloon dilation of narrowed vessels.

The scope of the present invention is defined in the attached claims.

## Claims

1. Angioplasty system, comprising:
- an inflation device (1) configured for the inflation of an angioplasty device within a blood vessel presenting stenosis, and comprising:
a reservoir (2) of contrast fluid including a pressure-adjusting device (3);
at least one connector (4, 5) for connection to the angioplasty device through disposable tubing connected to the reservoir (2);
a pressure sensor (6) within the reservoir (2);
an electronic actuation valve (7) for controlling the ingress or release of the contrast fluid;
safety means for the emergency deflation of the angioplasty device;
powering means of the inflation device (1);
a digital interface (8);
a microprocessor for controlling a number of operating parameters of the inflation device (1), operatively connected to the electronic actuation valve (7) and to the pressure-adjusting device (3), wherein the microprocessor comprises a first operation software with at least one operational application intended for the remote management of the inflation device (1);
a memory connected to the microprocessor; connection means of the microprocessor to the Internet or an intranet;
- a second artificial intelligence software for the automated self-learning of the inflation device (1), wherein the second software includes connection means thereof to the Internet and is operatively connected to the first operation software;
- a database (20) configured for the storage of data or treatment models and which is operatively connected to the Internet or an intranet and includes a management software; and
- a radiological arch (30) configured to acquire and process one or more images of the blood vessel or the area where the blood vessel is located;
wherein the microprocessor is operatively connected to the radiological arch (30) and to the database (20), in such a way that the inflation device (1) can perform a learning process to operate autonomously and regulate itself automatically, the system being configured for carrying out the following steps:
acquiring and processing, by means of the radiological arch (30), one or more images of the blood vessel or of an area where the blood vessel is located; and
operating and adjusting the inflation device (1) according to previous treatment models stored in the database, and treatment models obtained by running the second artificial intelligence software on, at least, data processed by the radiological arch (30) and on data from the inflation device (1) obtained by the first software.

2. System according to claim 1, further comprising heating means of the contrast fluid.

3. System according to any one of the previous claims, wherein the pressure adjustment device (3) comprises a motorised pump.

4. System according to any one of the previous claims, wherein the safety means comprise a vacuum reservoir (9) connected to the reservoir (2).

5. System according to claim 4, wherein the safety means further comprise manual safety means (10) for the release of the contrast fluid pressure.

6. System according to any one of the previous claims, wherein the disposable tubing connected to the reservoir (2) comprises two or more branches, each branch including a connector for connection to a different angioplasty device.

7. System according to any one of the previous claims, wherein the powering means comprise a battery or a connection to the electric grid.

8. System according to any one of the previous claims, wherein the connection means to the microprocessor comprise a connection interface installed on the inflation device (1).

9. System according to any one of the previous claims 1 to 8, wherein the connection means of the microprocessor comprise a connection to an external electronic device running a corresponding application.

10. System according to any one of the previous claims, wherein the digital interface (8) also comprises communication ports for data entry by means of a QR code reader or wirelessly.

## Patentansprüche

1. Angioplastiesystem, umfassend:
- eine Aufblasvorrichtung (1), welche zum Aufblasen einer Angioplastievorrichtung innerhalb eines Blutgefäßes, welches eine Stenose aufweist, ausgebildet ist, und umfassend:
einen Vorratsbehälter (2) von Kontrastflüssigkeit, welcher eine Druckeinstellvorrichtung (3) beinhaltet;
mindestens einen Verbinder (4, 5) zum Verbinden mit der Angioplastievorrichtung über einen Einwegschlauch, welcher mit dem Vorratsbehälter (2) verbunden ist;
einen Drucksensor (6) innerhalb des Vorratsbehälters (2);
ein elektronisches Betätigungsventil (7) zum Steuern des Zuflusses oder der Freisetzung der Kontrastflüssigkeit;
Sicherheitsmittel für das Notabblasen der Angioplastievorrichtung;
Stromversorgungsmittel der Aufblasvorrichtung (1);
eine digitale Schnittstelle (8);
einen Mikroprozessor zum Steuern einer Anzahl von Betriebsparametern der Aufblasvorrichtung (1), welcher mit dem elektronischen Betätigungsventil (7) und mit der Druckeinstellvorrichtung (3) betriebsmäßig verbunden ist, wobei der Mikroprozessor eine erste Betriebssoftware mit mindestens einer betrieblichen Anwendung, welche für die Fernverwaltung der Aufblasvorrichtung (1) vorgesehen ist, umfasst;
einen Speicher, welcher mit dem Mikroprozessor verbunden ist; Verbindungsmittel des Mikroprozessors mit dem Internet oder einem Intranet;
- eine zweite künstliche Intelligenz-Software für das automatisierte Selbstlernen der Aufblasvorrichtung (1), wobei die zweite Software Verbindungsmittel derselben mit dem Internet beinhaltet und mit der ersten Betriebssoftware betriebsmäßig verbunden ist;
- eine Datenbasis (20), welche für das Speichern von Daten oder Behandlungsmodellen ausgebildet ist und welche mit dem Internet oder einem Intranet betriebsmäßig verbunden ist und eine Verwaltungssoftware beinhaltet; und
- einen C-Bogen (30), welcher dazu ausgebildet ist, ein oder mehrere Bilder des Blutgefäßes oder des Bereichs, in welchem sich das Blutgefäß befindet, zu erfassen und zu verarbeiten;
wobei der Mikroprozessor derart mit dem C-Bogen (30) und mit der Datenbasis (20) betriebsmäßig verbunden ist, dass die Aufblasvorrichtung (1) einen Lernprozess durchführen kann, um selbstständig zu funktionieren und sich automatisch selbst zu regeln, wobei das System dazu ausgebildet ist, die folgenden Schritte vorzunehmen:
das Erfassen und das Verarbeiten, mittels des C-Bogens (30), eines oder mehrerer Bilder des Blutgefäßes oder eines Bereichs, in welchem sich das Blutgefäß befindet; und
das Betreiben und das Einstellen der Aufblasvorrichtung (1) gemäß den vorherigen Behandlungsmodellen, welche in der Datenbasis gespeichert sind, und Behandlungsmodellen, welche durch das Ausführen der zweiten künstlichen Intelligenz-Software erhalten wurden, auf, mindestens, vom C-Bogen (30) verarbeiteten Daten und auf Daten der Aufblasvorrichtung (1), welche durch die erste Software erhalten wurden.

2. System nach Anspruch 1, zusätzlich umfassend Heizmittel der Kontrastflüssigkeit.

3. System nach einem der vorhergehenden Ansprüche, wobei die Druckeinstellvorrichtung (3) eine motorisierte Pumpe umfasst.

4. System nach einem der vorhergehenden Ansprüche, wobei die Sicherheitsmittel einen Unterdruckvorratsbehälter (9) umfassen, welcher mit dem Vorratsbehälter (2) verbunden ist.

5. System nach Anspruch 4, wobei die Sicherheitsmittel zusätzlich manuelle Sicherheitsmittel (10) für die Entlastung des Kontrastflüssigkeitsdrucks umfassen.

6. System nach einem der vorhergehenden Ansprüche, wobei der Einwegschlauch, welcher mit dem Vorratsbehälter (2) verbunden ist, zwei oder mehr Verzweigungen umfasst, wobei jede Verzweigung einen Verbinder zum Verbinden mit einer unterschiedlichen Angioplastievorrichtung beinhaltet.

7. System nach einem der vorhergehenden Ansprüche, wobei die Stromversorgungsmittel eine Batterie oder eine Verbindung mit dem Elektrizitätsnetz umfassen.

8. System nach einem der vorhergehenden Ansprüche, wobei die Verbindungsmittel zum Mikroprozessor eine Verbindungsschnittstelle umfassen, welche auf der Aufblasvorrichtung (1) installiert ist.

9. System nach einem der vorhergehenden Ansprüche 1 bis 8, wobei die Verbindungsmittel des Mikroprozessors eine Verbindung mit einer äußeren elektronischen Vorrichtung umfassen, welche eine entsprechende Anwendung ausführt.

10. System nach einem der vorhergehenden Ansprüche, wobei die digitale Schnittstelle (8) auch Kommunikationsanschlüsse für die Dateneingabe mittels eines QR-Codelesers oder drahtlos umfasst.

## Revendications

1. Système d'angioplastie, comprenant :
- un dispositif de gonflage (1) configuré pour le gonflage d'un dispositif d'angioplastie à l'intérieur d'un vaisseau sanguin ayant une sténose, et comprenant :
un réservoir (2) de fluide de contraste comportant un dispositif de réglage de pression (3) ;
au moins un connecteur (4, 5) pour la connexion au dispositif d'angioplastie à travers une tubulure jetable connectée au réservoir (2) ;
un capteur de pression (6) à l'intérieur du réservoir (2) ;
une vanne d'actionnement électronique (7) pour contrôler l'entrée ou le dégagement du fluide de contraste ;
des moyens de sécurité pour le dégonflage d'urgence du dispositif d'angioplastie ;
des moyens d'alimentation de puissance du dispositif de gonflage (1) ;
une interface numérique (8) ;
un microprocesseur pour contrôler plusieurs paramètres de fonctionnement du dispositif de gonflage (1), connecté fonctionnellement à la vanne d'actionnement électronique (7) et au dispositif de réglage de pression (3), dans lequel le microprocesseur comprend un premier logiciel de fonctionnement avec au moins une application fonctionnelle destinée à la gestion à distance du dispositif de gonflage (1) ;
une mémoire connectée au microprocesseur ; des moyens de connexion du microprocesseur à Internet ou à un intranet ;
- un deuxième logiciel d'intelligence artificielle pour l'auto-apprentissage automatisé du dispositif de gonflage (1), dans lequel le deuxième logiciel comporte des moyens de connexion de celui-ci à Internet et il est connecté fonctionnellement au premier logiciel de fonctionnement ;
- une base de données (20) configurée pour le stockage de données ou de modèles de traitement et qui est connectée fonctionnellement à Internet ou à un intranet et comporte un logiciel de gestion ; et
- un arceau radiologique (30) configuré pour acquérir et traiter une ou plusieurs images du vaisseau sanguin ou de la zone où se trouve le vaisseau sanguin ;
dans lequel le microprocesseur est connecté fonctionnellement à l'arceau radiologique (30) et à la base de données (20), de telle sorte que le dispositif de gonflage (1) peut réaliser un procédé d'apprentissage pour fonctionner de manière autonome et se réguler automatiquement, le système étant configuré pour mettre en œuvre les étapes suivantes :
acquérir et traiter, par le biais de l'arceau radiologique (30), une ou plusieurs images du vaisseau sanguin ou d'une zone où se trouve le vaisseau sanguin ; et
faire fonctionner et réguler le dispositif de gonflage (1) selon des modèles de traitement précédents stockés dans la base de données, et des modèles de traitement obtenus par exécution du deuxième logiciel d'intelligence artificielle sur, au moins, des données traitées par l'arceau radiologique (30) et sur des données à partir du dispositif de gonflage (1) obtenues par le premier logiciel.

2. Système selon la revendication 1, comprenant en outre des moyens de chauffage du fluide de contraste.

3. Système selon l'une quelconque des revendications précédentes, dans lequel le dispositif de réglage de pression (3) comprend une pompe motorisée.

4. Système selon l'une quelconque des revendications précédentes, dans lequel les moyens de sécurité comprennent un réservoir à vide (9) connecté au réservoir (2).

5. Système selon la revendication 4, dans lequel les moyens de sécurité comprennent en outre des moyens de sécurité manuels (10) pour le dégagement de la pression de fluide de contraste.

6. Système selon l'une quelconque des revendications précédentes, dans lequel la tubulure jetable connectée au réservoir (2) comprend deux ou plusieurs branches, chaque branche comportant un connecteur pour la connexion à un dispositif d'angioplastie différent.

7. Système selon l'une quelconque des revendications précédentes, dans lequel les moyens d'alimentation de puissance comprennent une batterie ou une connexion au réseau électrique.

8. Système selon l'une quelconque des revendications précédentes, dans lequel les moyens de connexion au microprocesseur comprennent une interface de connexion installée sur le dispositif de gonflage (1).

9. Système selon l'une quelconque des revendications précédentes 1 à 8, dans lequel les moyens de connexion du microprocesseur comprennent une connexion à un dispositif électronique externe exécutant une application correspondante.

10. Système selon l'une quelconque des revendications précédentes, dans lequel l'interface numérique (8) comprend également des ports de communication pour l'entrée de données par le biais d'un lecteur de code QR ou de manière sans fil.
